# EUROPÄISCHE PATENTSCHRIFT

(11) **EP 3 073 937 B1**
(45) Veröffentlichungstag und Bekanntmachung des Hinweises auf die Patenterteilung: **27.09.2017**
(21) Anmeldenummer: 14796497.7
(22) Anmeldetag: 11.11.2014
(51) Int. Cl.: A61B 17/17, A61F 2/46, A61F 2/30, A61B 90/00, A61B 17/56

(54) **MEDIZINISCHES INSTRUMENTARIUM**
MEDICAL INSTRUMENT
ENSEMBLE D'INSTRUMENTS MÉDICAUX

(30) Priorität: 13.11.2013 DE 102013112496
(43) Veröffentlichungstag der Anmeldung: 05.10.2016
(73) Patentinhaber: Aesculap AG, 78532 Tuttlingen (DE)
(72) Erfinder: HERMLE, Thomas, 78628 Rottweil (DE); RICHTER, Berna, 78570 Mühlheim (DE)
(74) Vertreter: Hoeger, Stellrecht & Partner Patentanwälte mbB
(86) Internationale Anmeldenummer: PCT/EP2014/074252
(87) Internationale Veröffentlichungsnummer: WO 2015/071251

(56) Entgegenhaltungen:
- EP-A2- 2 168 507
- WO-A1-2012/010366
- US-A1- 2011 224 674

## Beschreibung

Die vorliegende Erfindung betrifft ein medizinisches Instrumentarium, insbesondere zum Implantieren einer Hüftgelenkpfanne, umfassend ein medizinisches Ausrichtinstrument mit einem Formkörper, welcher eine patientenindividuelle, vom Formkörper weg weisende und von einem Kugeloberflächenausschnitt abweichende Knochenanlagefläche aufweist, welche korrespondierend zu einer Knochenoberfläche des Patienten geformt ist, wobei der Formkörper mindestens eine Ansichtskante mit einer Ansichtshöhe aufweist, wobei die Ansichtskante an die patientenindividuelle Knochenanlagefläche angrenzt und wobei ein Übergang zwischen der Ansichtskante und der patientenindividuellen Knochenanlagefläche tangentenunstetig ist.

Für den Erfolg bei der Behandlung eines Patienten mit einer geschädigten Hüfte ist insbesondere die optimale Positionierung der Hüftgelenkpfanne der zu implantierenden Hüftgelenkendoprothese von Bedeutung. Hierzu ist es beispielsweise bekannt, Landmarken am Beckenknochen zu ertasten und hierüber eine Referenzebene des Beckens zu bestimmen, bezüglich derer alle Teile der Hüftgelenkendoprothese ausgerichtet und implantiert werden.

Insbesondere bei adipösen Patienten ist das Ertasten von Landmarken fehleranfällig, da der Knochen unter dem Gewebe nicht immer eindeutig zu tasten ist. Ein anderes Problem bei der Ertastung von Landmarken ist, dass diese gut erreichbar sein müssen. Dies ist insbesondere bei einem operativen Eingriff in Seitenlage nicht gegeben.

Ferner ist es bekannt, Referenzierungsvorrichtungen in Verbindung mit Navigationssystemen zu nutzen, um insbesondere die Hüftgelenkpfanne und den Hüftschaft in definierter Weise zu implantieren. Allerdings ist deren Einsatz durchaus zeitaufwendig.

In der US 2011/0224674 A1 sind patientenspezifische Ausrichtinstrumente für das Acetabulum beschrieben. Aus der WO 2012/010366 A1 sowie aus der EP 2 168 507 A2 sind Führungsinstrumente für Acetabulumfräser bekannt.

Es ist daher eine Aufgabe der vorliegenden Erfindung, die Implantation insbesondere einer Hüftgelenkpfanne einer Hüftgelenkendoprothese zu vereinfachen.

Diese Aufgabe wird bei einem medizinischen Instrumentarium der eingangs beschriebenen Art erfindungsgemäß dadurch gelöst, dass der Formkörper schalenförmig ausgebildet ist und eine konstante oder im Wesentlichen konstante Dicke aufweist und dass die Knochenanlagefläche eine Kontur aufweist, welche mindestens einem Teil der Fossa und/oder einem Teil der Incisura Acetabuli entspricht.

Die erfindungsgemäß vorgeschlagene Lösung vermeidet es, dass das Ausrichtinstrument mit der Knochenanlagefläche nicht vollständig an die Knochenfläche, beispielsweise einen Bereich des Acetabulums des Patienten, angelegt wird. Ist die Knochenanlagefläche insbesondere so groß, dass das gesamte Acetabulum als Referenz verwendet wird, hat der Arzt keine Sicht mehr in das Acetabulum hinein und damit auch keine Orientierung mehr. Er muss sich dann ausschließlich auf sein manuelles Gefühl verlassen, ob der Formkörper richtig, also flächig, im Acetabulum sitzt. Dies ist bei einer relativ großen Auflagefläche besonders schwierig zu erfühlen. Zudem steigt bei einer großen Auflagefläche das Risiko, dass Ungenauigkeiten an der patientenindividuellen Knochenanlagefläche oder Fremdkörper im Operationssitus einen eindeutigen und präzisen Sitz des Formkörpers am Acetabulum verhindern. Ein fehlpositionierter Formkörper bedeutet jedoch, dass der Operateur eine falsche Referenz für die Implantation verwendet, was zu einer erheblichen Fehlpositionierung des Implantats führen kann. Die erfindungsgemäß vorgeschlagene Lösung verhindert genau diese Probleme. Die in der angegebenen Weise ausgebildete Ansichtskante ermöglicht es, dass der Operateur zumindest eine eingeschränkte Sicht in das Acetabulum behält und so die Referenz während des Einsatzes des Ausrichtinstruments nicht nur manuell, sondern auch optisch kontrollieren kann. Dadurch, dass die Ansichtskante an die patientenindividuelle Knochenanlagefläche angrenzt, kann der Arzt das optimale Anliegen des Formkörpers am Acetabulum nicht nur fühlen, sondern auch sehen. Insbesondere ermöglicht es das Angrenzen der Ansichtskante an die patientenindividuelle Knochenanlagefläche, dass der Operateur eindeutig erkennen und insbesondere auch mit Tastinstrumenten, beispielsweise einen Tasthaken, prüfen kann, ob der Formkörper mit der patientenindividuellen Knochenanlagefläche in der vorbestimmten Weise im Acetabulum sitzt. Ist dies der Fall, dann kann das Ausrichtinstrument insbesondere genutzt werden, um durch Setzen von Referenzpins oder durch Referenzierung mittels eines Navigationssystems die Lage des Beckens des Patienten eindeutig zu bestimmen. Aufgrund der Eindeutigkeit der Knochenanlagefläche, die vorzugsweise auf Basis nichtinvasiv bestimmter Knochenkonturdaten des Patienten hergestellt wird, ergibt sich so eine einfache mechanische Ausrichthilfe, um die Position nicht nur des Beckens, sondern insbesondere des Acetabulums, zu bestimmen und zu referenzieren. Der Formkörper kann insbesondere zwei, drei, vier oder noch mehr Ansichtskanten aufweisen. Diese können zumindest teilweise parallel oder unter einem definierten Winkel relativ zueinander verlaufen. Möglich sind insbesondere auch in sich geschlossene, ringförmige Ansichtskanten, beispielsweise in Form innerer Begrenzungsflächen von Durchbrechungen des Formkörpers. Günstig ist es, dass der Formkörper schalenförmig ausgebildet ist und eine konstante oder im Wesentlichen konstante Dicke aufweist. So lässt er sich besonders einfach herstellen, beispielsweise durch Gießen oder 3D-Drucken. Außerdem ist er so leicht und nimmt nur ein kleines Volumen im Bereich des Operationssitus ein. Zudem ist auch eine Breite beziehungsweise Höhe der Ansichtskante nicht zu groß, was insbesondere eine Sicht auf den Übergang zwischen der Ansichtskante und der patientenindividuellen Knochenanlagefläche erschweren könnte. Ferner weist die Knochenanlagefläche eine Kontur auf, welche mindestens einem Teil der Fossa und/oder einem Teil der Incisura Acetabuli entspricht. Insbesondere die beiden genannten Strukturen, also die Fossa und die Incisura Acetabuli, ermöglichen patientenindividuell eine optimale Referenz für die Ausrichtung der zu implantierenden Hüftgelenkpfanne am Beckenknochen des Patienten. Mit dieser einfachen mechanischen, eindeutigen Ausrichthilfe lassen sich dann optional Ausricht- oder Befestigungspins positionieren und am Beckenknochen festlegen oder aber auch die Position des Ausrichtinstruments, insbesondere des Formkörpers, mit Hilfe eines Navigationssystems, beispielsweise unter Einsatz eines navigierten Tastinstruments, referenzieren.

Eine besonders sichere Überprüfung der genauen Position des Formkörpers im Acetabulum wird insbesondere dadurch möglich, dass die mindestens eine Ansichtskante eine Breite oder Höhe aufweist, welche mindestens einer minimalen Dicke des Formkörpers entspricht.

Für die Stabilität und insbesondere Ertastbarkeit der Ansichtskante ist es günstig, wenn die mindestens eine Ansichtskante eine Breite oder Höhe aufweist, welche maximal einem dreifachen einer minimalen Dicke des Formkörpers entspricht.

Gemäß einer weiteren bevorzugten Ausführungsform der Erfindung kann vorgesehen sein, dass die mindestens eine Ansichtskante einen ebenen oder im Wesentlichen ebenen Flächenbereich bildet oder eine Begrenzungsfläche einer Ausnehmung des Formkörpers bildet, welche Ausnehmung die Knochenanlagefläche mindestens teilweise durchdringt. Beispielsweise kann die Knochenanlagefläche in distaler Richtung weisend vom Formkörper weg weisen. Die Ausnehmung kann dann insbesondere seitlich am Formkörper ausgebildet sein und sich bis zur patientenindividuellen Knochenanlagefläche erstrecken.

Der Formkörper kann ferner einen Rand und optional auch einen Flächenbereich aufweisen, der nicht patientenindividuell geformt ist. Beispielsweise kann dieser Flächenbereich einen Ausschnitt einer Oberfläche eines Ellipsoids, insbesondere einer Kugel, definieren. Dieser Flächenbereich kann beispielsweise an einem Träger ausgebildet sein, welcher einen Teil des Formkörpers bildet, an welchem die patientenindividuelle Knochenanlagefläche an einem in distaler Richtung weisenden Vorsprung ausgebildet ist.

Günstig ist es, wenn die mindestens eine Ausnehmung in Form einer Durchbrechung ausgebildet ist und wenn die mindestens eine Ansichtskante eine Innenfläche der Durchbrechung bildet. Es können also insbesondere eine, zwei, drei, vier oder mehr Durchbrechungen am Formkörper vorgesehen sein, die es dem Operateur ermöglichen durch den Formkörper hindurch das Acetabulum zu sehen und insbesondere aufgrund der direkten Sicht auf die die Durchbrechungen begrenzenden Ansichtskanten optisch zu prüfen, ob der Formkörper in gewünschter Weise am Acetabulum anliegt oder nicht.

Vorteilhaft ist es, wenn das Ausrichtinstrument eine Instrumentenlängsachse definiert und wenn eine Längsachse der Durchbrechung parallel oder im Wesentlichen parallel zur Instrumentenlängsachse verläuft. So wird dem Operateur ein optimaler optischer Zugang zum Acetabulum durch den Formkörper hindurch ermöglicht.

Auf besonders einfache Weise lässt sich die Durchbrechung herstellen, wenn sie in Form einer Bohrung ausgebildet ist.

Um die Position des Formkörpers am Acetabulum unabhängig von einem Zugang und einer konkreten operativen Situation bestmöglich überprüfen zu können, ist es vorteilhaft, wenn das Ausrichtinstrument zwei, drei, vier oder mehr Ausnehmungen aufweist. Denkbar sind insbesondere beliebige Kombinationen ebener oder im Wesentlichen ebener Ausrichtkanten mit Ausnehmungen, insbesondere in Form von Durchbrechungen.

Ferner ist es vorteilhaft, wenn die Knochenanlagefläche mindestens teilweise an eine Kontur des Acetabulums des Patienten angepasst ist. So kann das Ausrichtinstrument eindeutig am Acetabulum positioniert werden, da dieses normalerweise eine von einer Rotationssymmetrie abweichende Form aufweist, die das Anlegen einer an die Knochenkontur angepasste patientenindividuelle Knochenanlagefläche nur in genau einer definierten Position ermöglicht.

Günstig ist es, wenn am Formkörper mindestens eine Befestigungselementaufnahme angeordnet oder ausgebildet ist. Es können insbesondere zwei, drei oder mehr Befestigungselementaufnahmen vorgesehen sein. Diese sind insbesondere korrespondierend zu Befestigungspins ausgebildet, die optional der Festlegung des Formkörpers am Beckenknochen dienen können oder aber auch der Festlegung einer Referenzierungsvorrichtung mit entsprechenden Markerelementen zur Referenzierung des Beckens mit Hilfe eines chirurgischen Navigationssystems.

Vorteilhaft ist es, wenn die Knochenanlagefläche und/oder der Formkörper durch Gießen oder 3D-Drucken hergestellt sind. So lässt sich der Formkörper auf einfache und kostengünstige Weise insbesondere aus einem Kunststoff herstellen, vorzugsweise einem sterilisierbaren Kunststoff. Optional kann der Formkörper beziehungsweise die Knochenanlagefläche auch aus einem Metall hergestellt werden.

Gemäß einer weiteren bevorzugten Ausführungsform der Erfindung kann vorgesehen sein, dass die Knochenanlagefläche des Formkörpers Knochenanlageflächenkonturdaten definiert, welche nichtinvasiv bestimmten Knochenkonturdaten des Patienten entsprechen oder im Wesentlichen entsprechen. Insbesondere kann es sich bei den Knochenkonturdaten des Patienten um Daten aus Röntgen- oder Magnetresonanzaufnahmen handeln. Diese können insbesondere durch entsprechende Röntgen- und Magnetresonanzgeräte bereits in digitaler Form bestimmt und dann direkt weiter verarbeitet werden zu Druckdaten oder für eine CAD-gesteuerte Fräse, zur Präparierung einer Gießform oder zum direkten Drucken der Knochenanlagefläche beziehungsweise des Formkörpers mit einem 3D-Drucker.

Besonders einfach handhaben lässt sich das Ausrichtinstrument, wenn es ein Griffelement umfasst.

Günstigerweise ist das Griffelement lösbar verbindbar mit dem Formkörper ausgebildet ist. So kann der Formkörper beispielweise mit dem Griffelement am Beckenknochen positioniert und dort mit Befestigungselementen, beispielsweise Knochenpins oder Knochenschrauben, temporär festgelegt werden. Um die Sicht auf den Operationssitus zu verbessern, kann dann das Griffelement optional vom Formkörper gelöst werden.

Auf besonders einfache Weise lassen sich das Griffelement und der Formkörper miteinander verbinden und auch voneinander lösen, wenn das Ausrichtinstrument eine Kopplungseinrichtung zum temporären Koppeln des Formkörpers mit dem Griffelement aufweist.

Auf besonders einfache und sichere Weise lassen sich das Griffelement und der Formkörper miteinander verbinden, wenn die Kopplungseinrichtung mindestens ein erstes Kopplungselement und mindestens ein zweites Kopplungselement umfasst, wenn die mindestens einen ersten und zweiten Kopplungselemente korrespondierend zueinander ausgebildet sind und in einer Kopplungsstellung in Eingriff stehen und in einer Lösestellung außer Eingriff stehen.

Das Verbinden des Griffelements mit dem Formkörper wird besonders einfach, insbesondere auch dann, wenn keine direkte Sicht auf den Operationssitus gegeben ist, wenn das mindestens eine erste Kopplungselement in Form einer Kopplungsaufnahme und wenn das mindestens eine zweite Kopplungselement in Form eines Kopplungsvorsprungs ausgebildet sind. Die Kopplungsaufnahme kann wahlweise am Griffelement oder auch am Formkörper ausgebildet sein.

Um eine möglichst hohe Stabilität bei der Verbindung der beiden Kopplungselemente in einer Kopplungsstellung zu erreichen, ist es vorteilhaft, wenn das mindestens eine erste Kopplungselement einen Innengewindeabschnitt und wenn das mindestens eine zweite Kopplungselement einen korrespondierenden Außengewindeabschnitt umfassen. Diese Ausgestaltung ermöglicht es insbesondere, das Griffelement an den Formkörper anzuschrauben oder von diesem abzuschrauben.

Vorzugsweise ist eines der beiden Kopplungselemente am Formkörper und das andere der beiden Kopplungselemente am Griffelement angeordnet oder ausgebildet. Insbesondere kann das mindestens eine erste Kopplungselement am Formkörper angeordnet oder ausgebildet sein und das mindestens eine zweite Kopplungselement am Griffelement.

Die Handhabung des Ausrichtungsinstruments lässt sich weiter verbessern, wenn das Griffelement einen langgestreckten Schaft umfasst und wenn an einem proximalen Endbereich des Schafts ein Griffbereich angeordnet oder ausgebildet ist. So kann ein Operateur das Griffelement am Griffbereich einfach und sicher halten. Beispielsweise kann der Griffbereich ergonomisch gestaltet sein.

Ferner ist es vorteilhaft, wenn der Schaft eine Längsachse definiert und wenn eine proximale Endfläche des Schafts in Form einer Schlagfläche für ein Einschlagwerkzeug ausgebildet ist. So ist es möglich, das Griffelement insbesondere zum Einschlagen beispielsweise des Formkörpers in das Acetabulum zu nutzen.

Günstigerweise ist die Schlagfläche mindestens teilweise eben und definiert eine senkrecht zur Längsachse verlaufende Ebene. So kann einfach und sicher ein Einschlagimpuls mit einem Einschlagwerkzeug auf das Griffelement ausgeübt werden, und zwar mit einer minimalen Gefahr des Abrutschens des Einschlagwerkzeugs von der Schlagfläche.

Ferner ist es vorteilhaft, wenn der Schaft mindestens zweiteilig ausgebildet ist und eine Schafthülse und einen in dieser geführten Innenschaft umfasst und wenn das zweite Kopplungselement an einem distalen Ende des Innenschafts oder an einem distalen Ende der Schafthülse angeordnet oder ausgebildet ist. Insbesondere kann der Innenschaft rotierbar und/oder verschiebbar in der Schafthülse gelagert und mit einem Betätigungselement, beispielsweise einem Drehknopf, gekoppelt sein. Dies ermöglicht es dem Operateur, beispielsweise das Griffelement am Griffbereich zu halten, der insbesondere mit der Schafthülse drehfest verbunden oder als Teil derselben ausgebildet sein kann, und dann den Innenschaft relativ zur Schafthülse zu verdrehen. Beispielsweise kann so der Innenschaft, wenn sein distales Ende mit einem Außengewindeabschnitt versehen ist, in eine entsprechende Kopplungsaufnahme mit Innengewinde am Formkörper eingeschraubt werden. Hierzu bedarf es lediglich der Rotation beispielsweise des Innenschafts, nicht jedoch des Griffelements beziehungsweise der Schafthülse als Ganzes.

Um den Formkörper mit einer definierten Kraft in das Acetabulum zu drücken, ist es günstig, wenn das Instrumentarium ferner ein Einschlagwerkzeug umfasst.

Die nachfolgende Beschreibung bevorzugter Ausführungsformen der Erfindung dient im Zusammenhang mit den Zeichnungen der näheren Erläuterung. Es zeigen:
- Figur 1:: eine schematische perspektivische Teilansicht des Beckens mit angelegtem Ausrichtinstrument am Acetabulum;
- Figur 2:: eine Explosionsdarstellung der Anordnung aus Figur 1;
- Figur 3:: eine Schnittansicht längs Linie 3-3 in Figur 1;

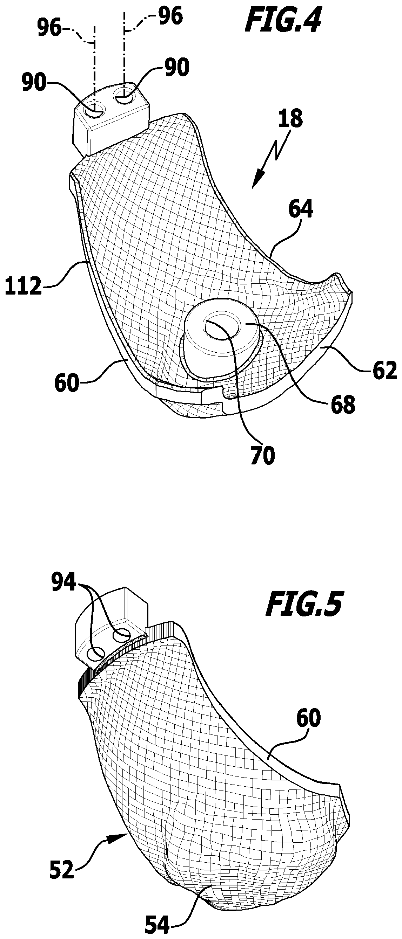

- Figur 4:: eine perspektivische Ansicht des Formkörpers des Ausrichtinstruments von proximal;
- Figur 5:: eine perspektivische Ansicht des Formkörpers aus Figur 4 von distal;
- Figur 6:: eine schematische perspektivische Ansicht ähnlich Figur 1 mit einem zweiten Ausführungsbeispiel eines Formkörpers;
- Figur 7:: eine schematische Explosionsdarstellung der Anordnung aus Figur 6;
- Figur 8:: eine Schnittansicht längs Linie 8-8 in Figur 6;
- Figur 9:: eine schematische perspektivische Ansicht des Formkörpers aus Figur 7 von proximal;
- Figur 10:: eine schematische perspektivische Ansicht des Formkörpers aus Figur 9 von distal;
- Figur 11:: eine schematische perspektivische Ansicht ähnlich Figur 1 mit einem weiteren Ausführungsbeispiel eines Formkörpers;
- Figur 12:: eine schematische Explosionsdarstellung der Anordnung aus Figur 11;
- Figur 13:: eine Schnittansicht längs Linie 13-13 in Figur 11;
- Figur 14:: eine schematische perspektivische Ansicht des Formkörpers aus Figur 12 von proximal;
- Figur 15:: eine schematische perspektivische Ansicht des Formkörpers aus Figur 14 von distal;

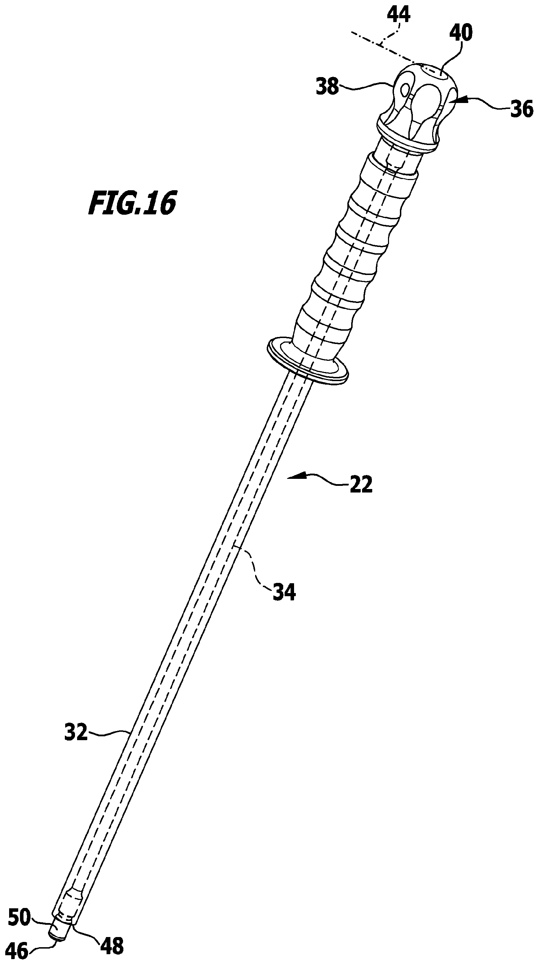

- Figur 16:: eine teilweise durchbrochene Ansicht des Griffelements aus den Figuren 2, 7 und 12.

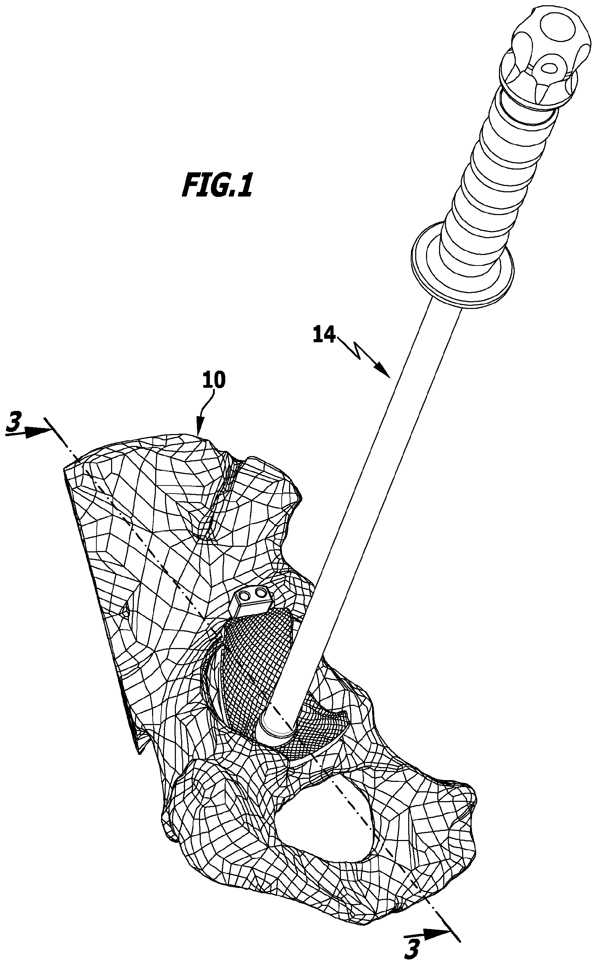

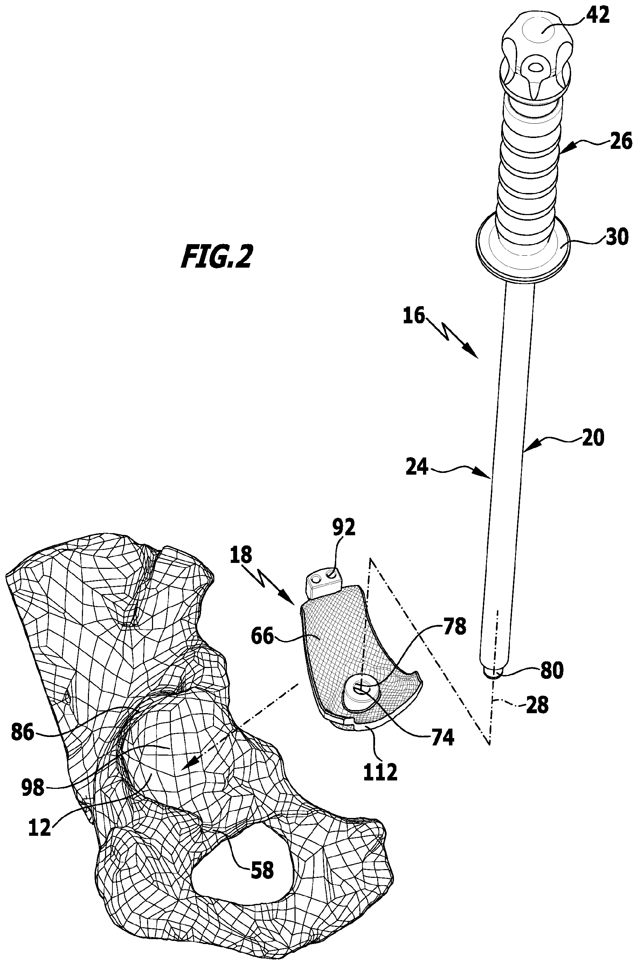

In Figur 1 ist schematisch ein Ausschnitt eines menschlichen Beckenknochens 10 eingezeichnet, in welchen eine Hüftgelenkendoprothese implantiert werden soll. Insbesondere ist in Figur 2 ein Acetabulum 12 dargestellt, welches auch als Hüftgelenk- oder Beckenpfanne bezeichnet wird und den in der Anatomie vom Beckenknochen 10 gebildeten knöchernen Anteil des natürlichen Hüftgelenks bildet.

Zur positionsgenauen Ausrichtung einer in den Figuren nicht dargestellten künstlichen Gelenkpfanne einer Hüftgelenkendoprothese dient ein insgesamt mit dem Bezugszeichen 14 bezeichnetes medizinisches Instrumentarium. Dieses umfasst insbesondere ein medizinisches Ausrichtinstrument 16 mit einem Formkörper 18 und einem Griffelement 20.

Das Griffelement 20 kann insbesondere als Einschläger 22 genutzt werden. Es umfasst einen langgestreckten Schaft 24, an dessen proximalem Ende ein Griffbereich 26 angeordnet oder ausgebildet ist. Ein in radialer Richtung bezogen auf eine Längsachse 28 abstehender Ringflansch 30 begrenzt den Griffbereich 26 distalseitig und dient insbesondere dazu, das Abrutschen einer Hand des das Griffelement 20 haltenden Operateurs in distaler Richtung zu verhindern.

Der Schaft 24 umfasst eine langgestreckte Schafthülse 32, die mit einer sich koaxial zur Längsachse 28 erstreckenden Längsbohrung versehen ist. In dieser ist ein langgestreckter Innenschaft 34 rotierbar und/oder verschiebbar gelagert. Ein proximales Ende des Innenschafts 34 ist mit einem Betätigungsglied 36 in Form eines Drehknopfs 38 drehfest gekoppelt.

Eine in proximaler Richtung weisende Endfläche 40 des Drehknopfs 38 bildet eine mindestens teilweise ebene Schlagfläche 42, welche eine Ebene 44 definiert, die senkrecht zur Längsachse 28 verläuft. Ein distales Ende 46 des Innenschafts 34 ragt über ein distales Ende 48 der Schafthülse 32 in distaler Richtung weisend vor. Ausgehend vom Ende 46 ist am Innenschaft 34 ein kurzer Außengewindeabschnitt 50 ausgebildet. Infolge einer Drehung des Drehknopfs 38 wird auch der Innenschaft 34 relativ zur Schafthülse 32 um die Längsachse 28 verdreht.

Der Formkörper 18 ist im Wesentlichen schalenförmig ausgebildet und weist eine nahezu konstante Wanddicke auf. Auf seiner in distaler Richtung weisenden Unterseite 52 weist der Formkörper 18 eine patientenindividuellen Knochenanlagefläche 54 auf. Diese korrespondiert zum Acetabulum 12 beziehungsweise zumindest zu der für jeden Patienten spezifischen Fossa 56 und/oder zur Incisura Acetabuli 58. Die Knochenanlagefläche 54 weicht von einem Kugeloberflächenausschnitt ab.

Die Knochenanlagefläche 54 am Formkörper 18 und/oder der Formkörper 18 sind vorzugsweise durch Gießen oder 3D-Drucken hergestellt. Zum Gießen wird beispielsweise eine Gießform auf Basis nichtinvasiv bestimmter Knochenkonturdaten des Patienten ausgebildet. Zum Beispiel können die Knochenkonturdaten aus Röntgen- und/oder Magnetresonanzaufnahmen des Acetabulums 42 des Patienten stammen. Beispielsweise können direkt digitalisierte Röntgenaufnahmen weiterverarbeitet werden zu Druckdaten für einen 3D-Drucker, mit dem dann die Knochenanlagefläche 54 auf einem Träger beziehungsweise als Teil des Formkörpers 18 mit diesem zusammen gedruckt werden kann. Auf diese Weise kann dann eine Knochenanlagefläche 54 ausgebildet werden, die Knochenanlageflächenkonturdaten definiert, die den nichtinvasiv bestimmten Knochenkonturdaten des Patienten entsprechen.

Am Formkörper 18 sind ferner mehrere Ansichtskanten 60, 62 und 64 in Form von Ausnehmungen 112 oder Abflachungen als Flächenbereiche mit einer Ansichtshöhe ausgebildet, die an die patientenindividuelle Knochenanlagefläche 54 angrenzen. Ein Übergang zwischen den Ansichtskanten 60, 62 und 64 und der Knochenanlagefläche 54 ist tangentenunstetig. Die Ansichtskanten 60, 62 und 64 sind jeweils eben beziehungsweise im Wesentlichen eben ausgebildet. Die Ansichtskanten 60 und 64 verlaufen nahezu parallel zueinander.

Von einer in proximaler Richtung weisenden Oberseite 66 des Formkörpers ist ein Zapfen 68 in proximaler Richtung weisend ausgebildet. Er ist mit einem Sackloch 70 versehen, welches mit einem Innengewindeabschnitt 72 versehen ist. Der Innengewindeabschnitt 72 ist korrespondierend zum Außengewindeabschnitt 50 ausgebildet.

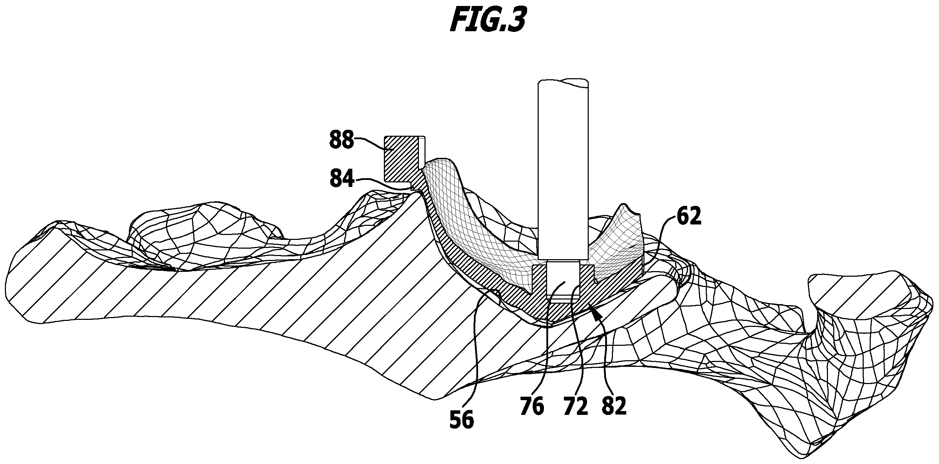

Das Sackloch 70 bildet eine Kopplungsaufnahme 74, die korrespondierend zu einem durch das Ende 46 gebildeten Kopplungsvorsprungs 76 ausgebildet ist. Die Kopplungsaufnahme 74 und der Kopplungsvorsprung 76 bilden erste und zweite Kopplungselemente 78 und 80 einer insgesamt mit dem Bezugszeichen 82 bezeichneten Kopplungseinrichtung zum temporären Koppeln des Formkörpers 18 mit dem Griffelement 20 in einer Kopplungsstellung wie sie beispielsweise in den Figuren 1 und 3 schematisch dargestellt ist.

Ferner ist am Formkörper 18 ein Randabschnitt 84 ausgebildet, welcher einen Rand 86 des Acetabulums 12 gegenüberliegend der Incisura Acetabuli übergreift.

Am Abschnitt 84 kann optional ein Befestigungskörper 88 angeordnet oder ausgebildet sein, welcher im Wesentlichen quaderförmig ausgebildet ist. Am Befestigungskörper 88 sind zwei Befestigungselementaufnahmen 90 ausgebildet in Form von Durchbrechungen 92, die als Bohrungen 94 hergestellt sind. Vorzugsweise verlaufen Befestigungselementaufnahmelängsachsen 96 parallel zur Längsachse 28.

Durch die Befestigungselementaufnahmen 90 können insbesondere Befestigungspins in den Beckenknochen 10 eingebracht werden, um zum einen das Ausrichtinstrument 16 am Acetabulum 12 zu fixieren und/oder um an ihnen Referenzierungsvorrichtungen mit Markerelementen festzulegen, um die Position des Formkörpers 18 am Beckenknochen 10 und damit dessen Lage im Raum mit Hilfe eines Navigationssystems zu bestimmen.

Wie insbesondere in den Figuren 1 und 3 gut zu erkennen, ermöglicht es das Vorsehen der Ansichtskanten 60, 62 und 64 einem Operateur, die positionsrichtige Anordnung des Formkörpers 18 im Acetabulum 12 auf Sicht oder mit Hilfe eines Tasthakens zu prüfen. Nur dann, wenn der Formkörper 18 mit der Knochenanlagefläche 54 flächig an der Knochenfläche 98 am Beckenknochen 10 anliegt, entspricht die Position und Ausrichtung des Formkörpers 18 den insbesondere nichtinvasiv bestimmten Knochenkonturdaten.

Mit Hilfe des Ausrichtinstruments 16 kann nun unter Einsatz weiterer Instrumente und Werkzeuge das Acetabulum 12 als Pfannenlager für die Gelenkpfanne der zu implantierenden Hüftgelenkendoprothese vorbereitet werden.

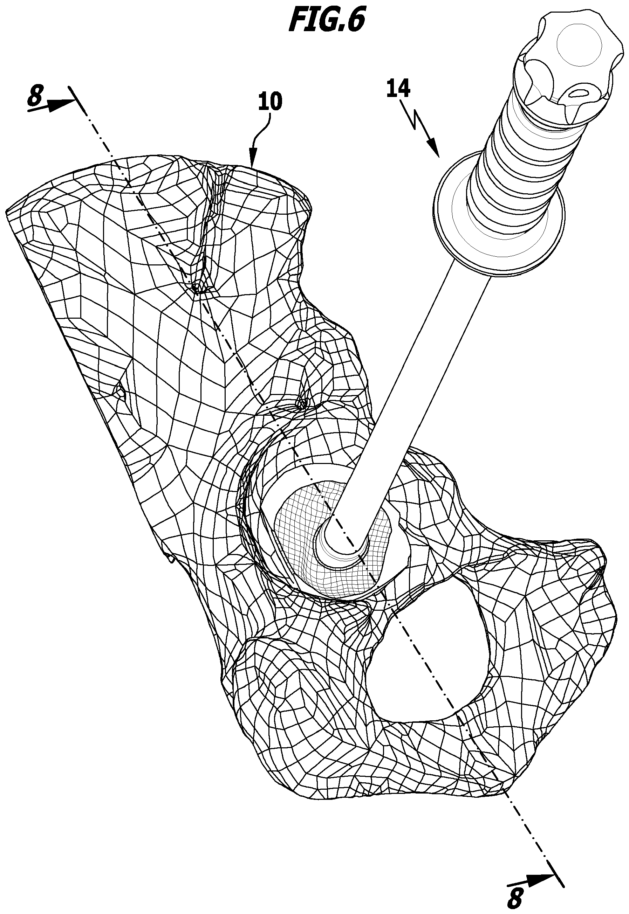

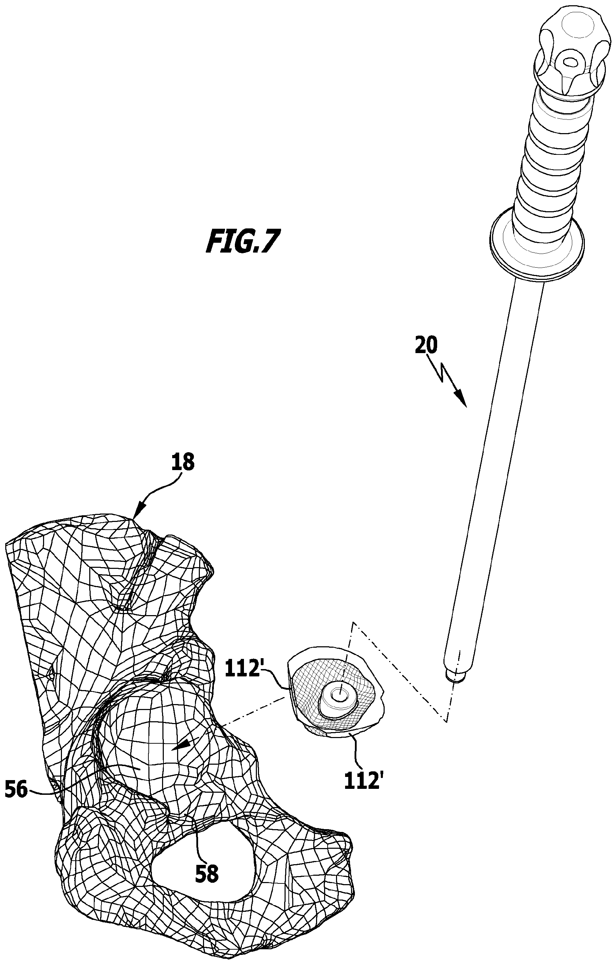

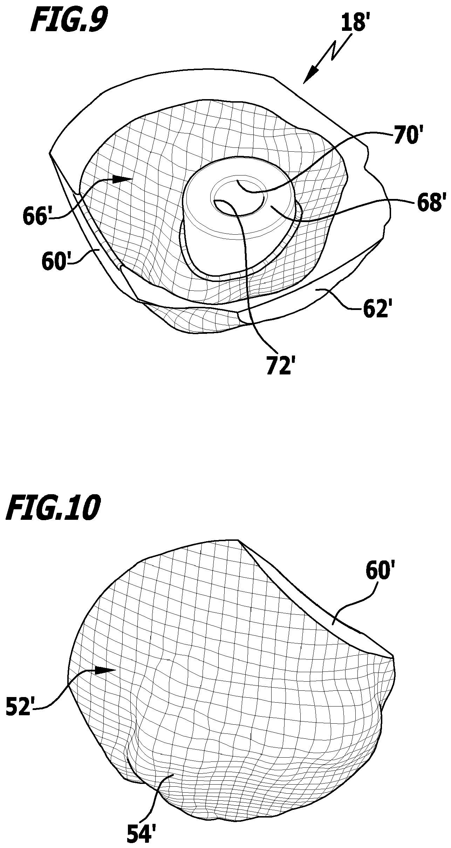

In den Figuren 6 bis 10 ist ein zweites Ausführungsbeispiel eines mit dem Bezugszeichen 18' bezeichneten Formkörpers dargestellt. Es bildet einen Teil des Instrumentariums 14. Der Übersichtlichkeit wegen sind identische Teile und Elemente des Formkörpers 18' mit entsprechenden Bezugszeichen versehen, die beim Formkörper 18 benutzt wurden.

Auch der Formkörper 18' ist schalenförmig ausgebildet und weist auf seiner Unterseite 52' eine Knochenanlagefläche 54' auf, die einem negativen Abbild der Fossa 56 entspricht. Ferner sind am Formkörper 18' Ausnehmungen 112' beziehungsweise Abflachungen in Form ebener Ansichtskanten 60' und 62' vorgesehen, die im Wesentlichen senkrecht zueinander orientiert sind und jeweils Ebenen definieren, die im Wesentlichen parallel zur Längsachse 28 verlaufen.

Auf einer Oberseite 66' des Formkörpers 18' steht ebenfalls ein Zapfen 68' ab, welcher mit einem Sackloch 70' versehen ist, das einen Innengewindeabschnitt 72' aufweist. So kann der Formkörper 18' in analoger Weise wie der Formkörper 18 mit dem Griffelement 20 in der oben beschriebenen Weise gekoppelt werden.

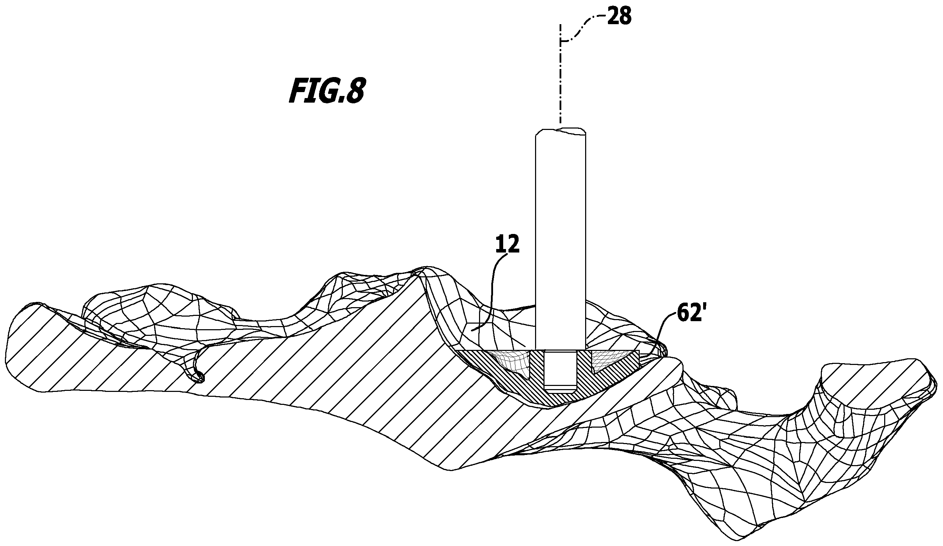

Wie insbesondere in den Figuren 6 und 8 gut zu erkennen, ermöglichen die Ansichtskanten 60' und 62' einem Operateur eine optische Prüfung oder eine Prüfung in Verbindung mit einem Tastinstrument, beispielsweise einem Tasthaken, ob der Formkörper 18' passgenau im Acetabulum 12 positioniert ist.

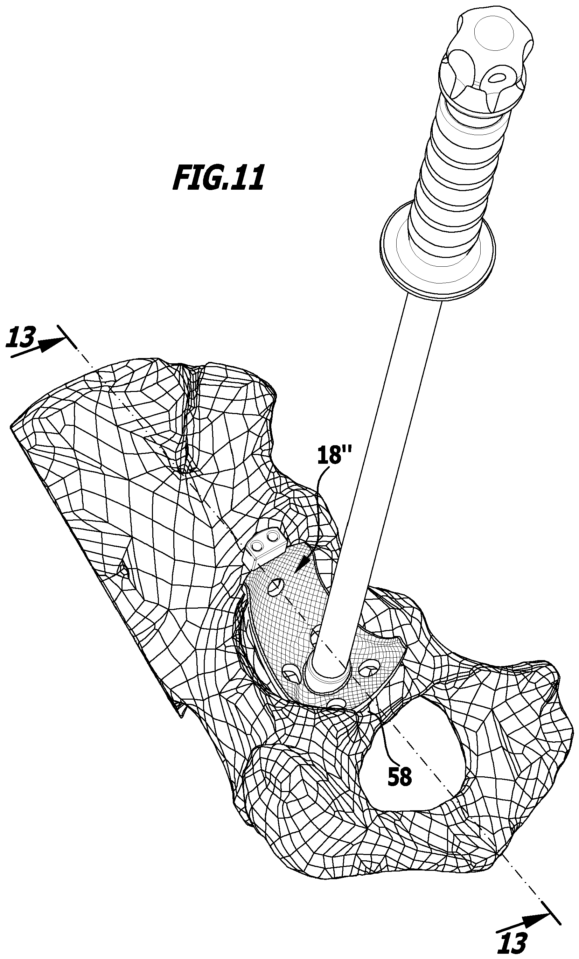

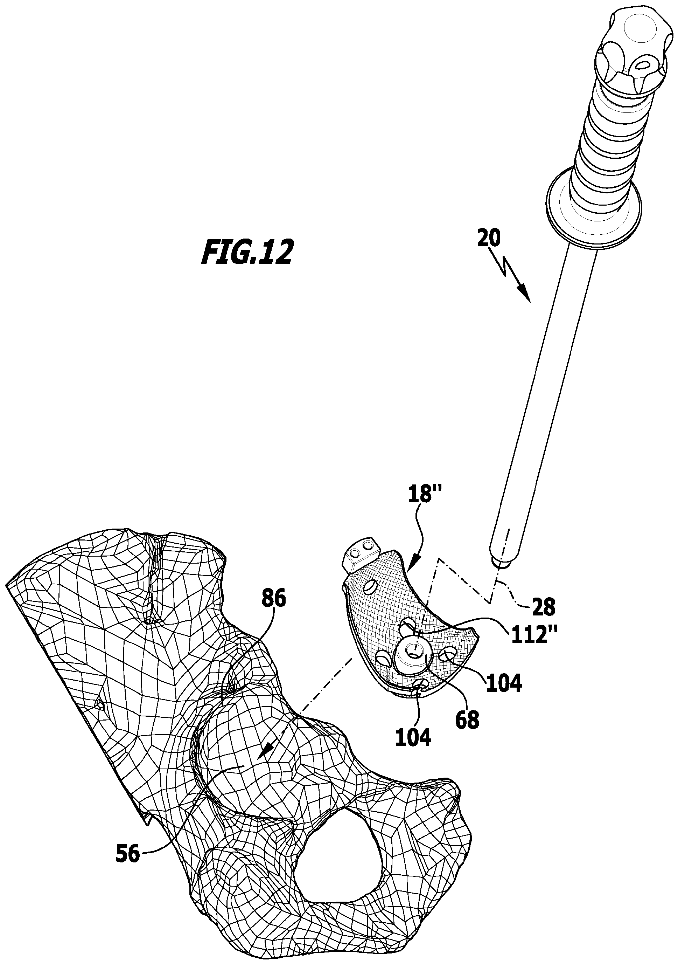

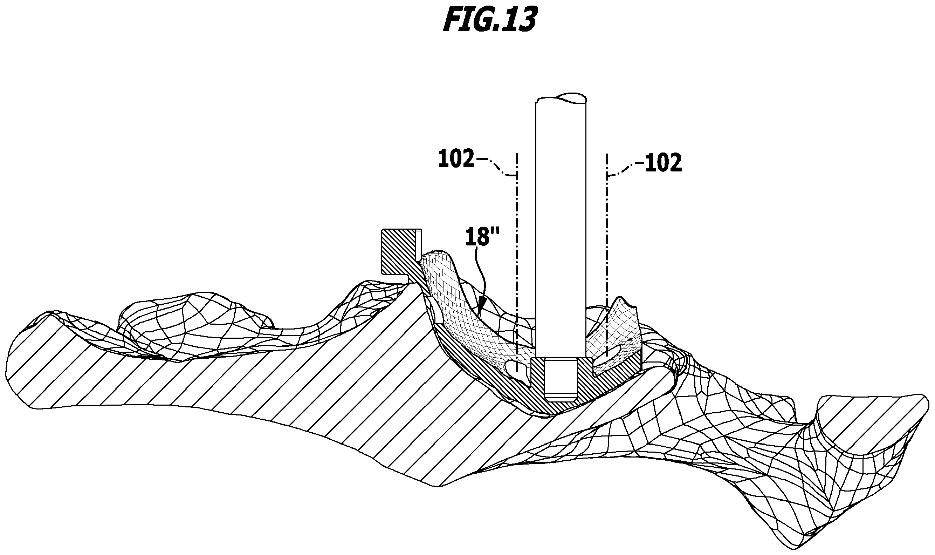

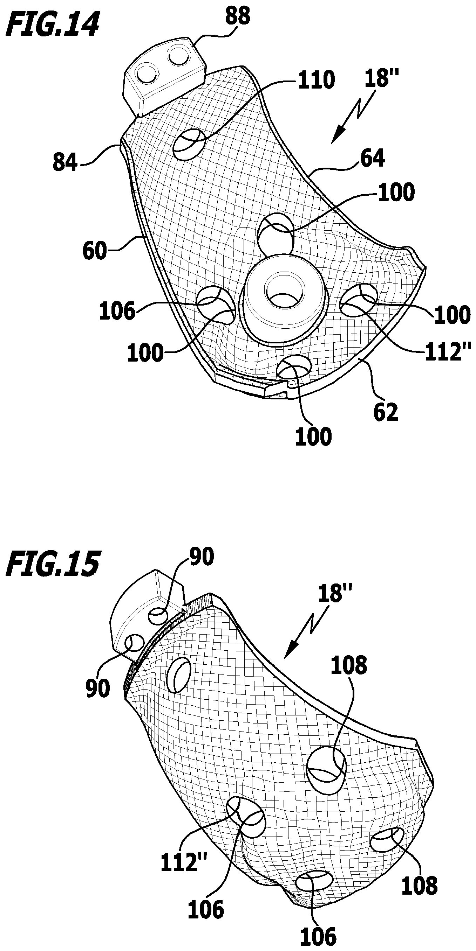

In den Figuren 11 bis 15 ist ein drittes Ausführungsbeispiel eines insgesamt mit dem Bezugszeichen 18" bezeichneten Formkörpers des Instrumentariums 14 schematisch dargestellt. Der Aufbau des Formkörpers 18" entspricht im Wesentlichen dem Aufbau des Formkörpers 18, so dass beim Formkörper 18" identische Bezugszeichen verwendet wurden wie beim Formkörper 18.

Der Formkörper 18" unterscheidet sich vom Formkörper 18' dadurch, dass er insgesamt vier im Wesentlichen gleichmäßig um den Zapfen 68 herum angeordnete Ausnehmungen 112" in Form von Durchbrechungen 100 aufweist. Die Durchbrechungen 100 definieren Durchbrechungslängsachsen 102, die im Wesentlichen parallel zur Längsachse 28 verlaufen. Die Durchbrechungen 100 sind in Form von Bohrungen 104 ausgebildet. Innenflächen 106 der Bohrungen 104 bilden Ansichtskanten 108, die einen direkten Blick auf das Acetabulum 12 ermöglichen. So können die Durchbrechungen 100 durch einen Operateur insbesondere als Sichtfenster genutzt werden, um die optimale Positionierung des Formkörpers 18" am Acetabulum 12 auf Sicht zu überprüfen.

Optional können am Formkörper 18" auch weitere Durchbrechungen 110 vorgesehen sein, die außerhalb der patientenindividuellen Knochenanlagefläche 54 in Form von Bohrungen ausgebildet sein können.

Die Formkörper 18, 18' und 18" können alle aus einem Kunststoff oder einem Metall durch Gießen oder 3D-Drucken hergestellt sein.

### Bezugszeichenliste

- 10:: Beckenknochen
- 12:: Acetabulum
- 14:: Instrumentarium
- 16:: Ausrichtinstrument
- 18, 18', 18":: Formkörper
- 20:: Griffelement
- 22:: Einschläger
- 24:: Schaft
- 26:: Griffbereich
- 28:: Längsachse
- 30:: Ringflansch
- 32:: Schafthülse
- 34:: Innenschaft
- 36:: Betätigungsglied
- 38:: Drehknopf
- 40:: Endfläche
- 42:: Schlagfläche
- 44:: Ebene
- 46:: Ende
- 48:: Ende
- 50:: Außengewindeabschnitt
- 52, 52':: Unterseite
- 54, 54':: Knochenanlagefläche
- 56:: Fossa
- 58:: Incisura Acetabuli
- 60, 60':: Ansichtskante
- 62, 62':: Ansichtskante
- 64:: Ansichtskante
- 66, 66':: Oberseite
- 68, 68':: Zapfen
- 70, 70':: Sackloch
- 72, 72':: Innengewindeabschnitt
- 74:: Kopplungsaufnahme
- 76:: Kopplungsvorsprung
- 78:: erstes Kopplungselement
- 80:: zweites Kopplungselement
- 82:: Kopplungseinrichtung
- 84:: Randabschnitt
- 86:: Rand
- 88:: Befestigungskörper
- 90:: Befestigungselementaufnahme
- 92:: Durchbrechungen
- 94:: Bohrung
- 96:: Befestigungselementaufnahmelängsachsen
- 98:: Knochenfläche
- 100:: Durchbrechung
- 102:: Durchbrechungslängsachse
- 104:: Bohrung
- 106:: Innenfläche
- 108:: Ansichtskante
- 110:: Durchbrechung
- 112, 112', 112":: Ausnehmung

## Patentansprüche

1. Medizinisches Instrumentarium (14), insbesondere zum Implantieren einer Hüftgelenkpfanne, umfassend ein medizinisches Ausrichtinstrument (16) mit einem Formkörper (18; 18'; 18"), welcher eine patientenindividuelle, vom Formkörper (18; 18'; 18") weg weisende und von einem Kugeloberflächenausschnitt abweichende Knochenanlagefläche (54; 54') aufweist, welche korrespondierend zu einer Knochenoberfläche des Patienten geformt ist, wobei der Formkörper (18; 18'; 18") mindestens eine Ansichtskante (60, 62, 64; 60', 62'; 108) mit einer Ansichtshöhe aufweist, wobei die Ansichtskante (60, 62, 64; 60', 62'; 108) an die patientenindividuelle Knochenanlagefläche (54; 54') angrenzt und wobei ein Übergang zwischen der Ansichtskante (60, 62, 64; 60', 62'; 108) und der patientenindividuellen Knochenanlagefläche (54; 54') tangentenunstetig ist, **dadurch gekennzeichnet, dass** der Formkörper (18; 18'; 18") schalenförmig ausgebildet ist und eine konstante oder im Wesentlichen konstante Dicke aufweist und dass die Knochenanlagefläche (54; 54') eine Kontur aufweist, welche mindestens einem Teil der Fossa (56) und/oder einem Teil der Incisura Acetabuli (58) entspricht.

2. Medizinisches Instrumentarium nach Anspruch 1, **dadurch gekennzeichnet, dass** die mindestens eine Ansichtskante (60, 62, 64; 60', 62'; 108) eine Breite oder Höhe aufweist, welche mindestens einer minimalen Dicke des Formkörpers (18; 18'; 18") entspricht.

3. Medizinisches Instrumentarium nach Anspruch 2, **dadurch gekennzeichnet, dass** die mindestens eine Ansichtskante (60, 62, 64; 60', 62'; 108) eine Breite oder Höhe aufweist, welche maximal einem dreifachen einer minimalen Dicke des Formkörpers (18; 18'; 18") entspricht.

4. Medizinisches Instrumentarium nach einem der voranstehenden Ansprüche, **dadurch gekennzeichnet, dass** die mindestens eine Ansichtskante (60, 62, 64; 60', 62'; 108) einen ebenen oder im Wesentlichen ebenen Flächenbereich bildet oder eine Begrenzungsfläche einer Ausnehmung (112; 112'; 112") des Formkörpers (18; 18'; 18") bildet, welche Ausnehmung (112; 112'; 112") die Knochenanlagefläche (54; 54') mindestens teilweise durchdringt.

5. Medizinisches Instrumentarium nach Anspruch 4, **dadurch gekennzeichnet, dass** die mindestens eine Ausnehmung (112; 112'; 112") in Form einer Durchbrechung (100) ausgebildet ist und dass die mindestens eine Ansichtskante (108) eine Innenfläche (106) der Durchbrechung (100) bildet.

6. Medizinisches Instrumentarium nach Anspruch 5, **dadurch gekennzeichnet,**
a) **dass** das Ausrichtinstrument (16) eine Instrumentenlängsachse (28) definiert und dass eine Längsachse (102) der Durchbrechung (100) parallel oder im Wesentlichen parallel zur Instrumentenlängsachse (28) verläuft
und/oder
b) **dass** die Durchbrechung (100) in Form einer Bohrung (104) ausgebildet ist.

7. Medizinisches Instrumentarium nach einem der voranstehenden Ansprüche, **dadurch gekennzeichnet,**
a) **dass** die Knochenanlagefläche (54; 54') mindestens teilweise an eine Kontur des Acetabulums (12) des Patienten angepasst ist und/oder
b) **dass** am Formkörper (18; 18") mindestens eine Befestigungselementaufnahme (90) angeordnet oder ausgebildet ist
und/oder
c) **dass** die Knochenanlagefläche (54; 54') und/oder der Formkörper durch Gießen oder 3D-Drucken hergestellt ist
und/oder
d) **dass** die Knochenanlagefläche (54; 54') des Formkörpers (18; 18'; 18") Knochenanlageflächenkonturdaten definiert, welche nichtinvasiv bestimmten Knochenkonturdaten des Patienten entsprechen oder im Wesentlichen entsprechen, insbesondere Knochenkonturdaten des Patienten aus Röntgen- oder Magnetresonanzaufnahmen.

8. Medizinisches Instrumentarium nach einem der voranstehenden Ansprüche, **gekennzeichnet durch** ein Griffelement (20).

9. Medizinisches Instrumentarium nach Anspruch 8, **dadurch gekennzeichnet, dass** das Griffelement (20) lösbar verbindbar mit dem Formkörper (18; 18'; 18") ausgebildet ist.

10. Medizinisches Instrumentarium nach einem der voranstehenden Ansprüche, **gekennzeichnet durch** eine Kopplungseinrichtung (82) zum temporären Koppeln des Formkörpers (18; 18'; 18") mit dem Griffelement (20).

11. Medizinisches Instrumentarium nach Anspruch 10, **dadurch gekennzeichnet, dass** die Kopplungseinrichtung (82) mindestens ein erstes Kopplungselement (78) und mindestens ein zweites Kopplungselement (80) umfasst, dass die mindestens einen ersten und zweiten Kopplungselemente (78, 80) korrespondierend zueinander ausgebildet sind und in einer Kopplungsstellung in Eingriff stehen und in einer Lösestellung außer Eingriff stehen.

12. Medizinisches Instrumentarium nach Anspruch 11, **dadurch gekennzeichnet,**
a) **dass** das mindestens eine erste Kopplungselement (78) in Form einer Kopplungsaufnahme (74) und das mindestens eine zweite Kopplungselement (80) in Form eines Kopplungsvorsprungs (76) ausgebildet sind
und/oder
b) **dass** das mindestens eine erste Kopplungselement (78) einen Innengewindeabschnitt (72) und das mindestens eine zweite Kopplungselement (80) einen korrespondierenden Außengewindeabschnitt (50) umfassen
und/oder
c) **dass** das mindestens eine erste Kopplungselement (78) am Formkörper (18; 18'; 18") angeordnet oder ausgebildet ist und dass das mindestens eine zweite Kopplungselement (80) am Griffelement (20) angeordnet oder ausgebildet ist.

13. Medizinisches Instrumentarium nach einem der Ansprüche 8 bis 12, **dadurch gekennzeichnet, dass** das Griffelement (20) einen langgestreckten Schaft (24) umfasst und dass an einem proximalen Endbereich des Schafts (24) ein Griffbereich (26) angeordnet oder ausgebildet ist.

14. Medizinisches Instrumentarium nach Anspruch 13, **dadurch gekennzeichnet,**
a) **dass** der Schaft (24) eine Längsachse (28) definiert und dass eine proximale Endfläche des Schafts (24) in Form einer Schlagfläche (42) für ein Einschlagwerkzeug ausgebildet ist
und/oder
b) **dass** der Schaft (24) mindestens zweiteilig ausgebildet ist und eine Schafthülse (32) und einen in dieser geführten Innenschaft (34) umfasst und dass das zweite Kopplungselement (80) an einem distalen Ende (46) des Innenschafts (34) oder an einem distalen Ende der Schafthülse angeordnet oder ausgebildet ist.

15. Medizinisches Instrumentarium nach einem der voranstehenden Ansprüche, **gekennzeichnet durch** ein Einschlagwerkzeug.

## Claims

1. Medical instrumentation (14), in particular, for implanting an acetabular cup, comprising a medical alignment instrument (16) with a shaped body (18; 18'; 18"), which has a patient-specific bone abutment surface (54; 54') facing away from the shaped body (18; 18'; 18") and deviating from a spherical surface section, the bone abutment surface being shaped so as to correspond to a bone surface of the patient, wherein the shaped body (18; 18'; 18") has at least one viewing edge (60, 62, 64; 60', 62'; 108) with a viewing height, wherein the viewing edge (60, 62, 64; 60', 62'; 108) adjoins the patient-specific bone abutment surface (54; 54'), and wherein a transition between the viewing edge (60, 62, 64; 60', 62'; 108) and the patient-specific bone abutment surface (54; 54') is tangentially discontinuous, **characterized in that** the shaped body (18; 18'; 18") is of dish-shaped construction and has a constant or substantially constant thickness and **in that** the bone abutment surface (54; 54') has a contour which corresponds at least to a portion of the fossa (56) and/or to a portion of the acetabular notch (58).

2. Medical instrumentation in accordance with claim 1, **characterized in that** the at least one viewing edge (60, 62, 64; 60', 62'; 108) has a width or height which corresponds at least to a minimum thickness of the shaped body (18; 18'; 18").

3. Medical instrumentation in accordance with claim 2, **characterized in that** the at least one viewing edge (60, 62, 64; 60', 62'; 108) has a width or height which corresponds at most to three times a minimum thickness of the shaped body (18; 18'; 18").

4. Medical instrumentation in accordance with any one of the preceding claims, **characterized in that** the at least one viewing edge (60, 62, 64; 60', 62'; 108) forms a flat or substantially flat surface area or forms a boundary surface of a recess (112; 112'; 112") of the shaped body (18; 18'; 18"), which recess (112; 112'; 112") at least partially penetrates the bone abutment surface (54; 54').

5. Medical instrumentation in accordance with claim 4, **characterized in that** the at least one recess (112; 112'; 112") is constructed in the form of a through-opening (100), and **in that** the at least one viewing edge (108) forms an inner surface (106) of the through-opening (100).

6. Medical instrumentation in accordance with claim 5, **characterized in that**
a) the alignment instrument (16) defines an instrument longitudinal axis (28), and **in that** a longitudinal axis (102) of the through-opening (100) runs parallel or substantially parallel to the instrument longitudinal axis (28)
and/or
b) **in that** the through-opening (100) is constructed in the form of a borehole (104).

7. Medical instrumentation in accordance with any one of the preceding claims, **characterized in that**
a) the bone abutment surface (54; 54') is at least partially adapted to a contour of the acetabulum (12) of the patient
and/or
b) **in that** at least one attachment element receptacle (90) is arranged or formed on the shaped body (18; 18")
and/or
c) **in that** the bone abutment surface (54; 54') and/or the shaped body are manufactured by molding or 3-D printing
and/or
d) **in that** the bone abutment surface (54; 54') of the shaped body (18; 18'; 18") defines bone abutment surface contour data which correspond or substantially correspond to noninvasively determined bone contour data of the patient, in particular, bone contour data of the patient from X-ray or magnetic resonance images.

8. Medical instrumentation in accordance with any one of the preceding claims, **characterized by** a grip element (20).

9. Medical instrumentation in accordance with claim 8, **characterized in that** the grip element (20) is constructed so as to be releasably connectable to the shaped body (18; 18'; 18").

10. Medical instrumentation in accordance with any one of the preceding claims, **characterized by** a coupling device (82) for temporarily coupling the shaped body (18; 18'; 18") to the grip element (20).

11. Medical instrumentation in accordance with claim 10, **characterized in that** the coupling device (82) comprises at least one first coupling element (78) and at least one second coupling element (80), and **in that** the at least one first and second coupling elements (78, 80) are constructed so that they correspond to each other and are in engagement in a coupling position and in disengagement in a release position.

12. Medical instrumentation in accordance with claim 11, **characterized in that**
a) the at least one first coupling element (78) is constructed in the form of a coupling receptacle (74) and the at least one second coupling element (80) in the form of a coupling projection (76) and/or
b) **in that** the at least one first coupling element (78) has an internal thread section (72) and the at least one second coupling element (80) has a corresponding external thread section (50)
and/or
c) **in that** the at least one first coupling element (78) is arranged or constructed on the shaped body (18; 18'; 18"), and **in that** the at least one second coupling element (80) is arranged or constructed on the grip element (20).

13. Medical instrumentation in accordance with any one of claims 8 to 12, **characterized in that** the grip element (20) has an elongate shaft (24), and **in that** a gripping area (26) is arranged or formed at a proximal end area of the shaft (24).

14. Medical instrumentation in accordance with claim 13, **characterized in that**
a) the shaft (24) defines a longitudinal axis (28), and **in that** a proximal end face of the shaft (24) is constructed in the form of an impact surface (42) for an impactor tool
and/or
b) **in that** the shaft (24) is of at least two-part construction and comprises a shaft sleeve (32) and an inner shaft (34) guided in the shaft sleeve, and **in that** the second coupling element (80) is arranged or formed at a distal end (46) of the inner shaft (34) or at a distal end of the shaft sleeve.

15. Medical instrumentation in accordance with any one of the preceding claims, **characterized by** an impactor tool.

## Revendications

1. Ensemble d'instruments médicaux (14), en particulier pour implanter une cuvette d'articulation de la hanche, comprenant un instrument d'alignement médical (16) avec un corps moulé (18 ; 18' ; 18") présentant une surface de contact des os (54 ; 54') adaptée au patient, faisant suite au corps moulé (18 ; 18' ; 18") et étant déviée d'un segment de surface sphérique et moulée en fonction d'une surface des os du patient, dans lequel le corps moulé (18 ; 18' ; 18") présente au moins un bord visible (60, 62, 64 ; 60', 62' ; 108) d'une certaine hauteur visible, dans lequel le bord visible (60, 62, 64 ; 60', 62' ; 108) est délimité par la surface de contact des os (54 ; 54') adaptée au patient et dans lequel un raccordement à continuité de tangente existe entre le bord visible (60, 62, 64 ; 60', 62' ; 108) et la surface de contact des os (54 ; 54') adaptée au patient, **caractérisé en ce que** le corps moulé (18 ; 18' ; 18") est en forme de coque et possède une épaisseur constante ou sensiblement constante et **en ce que** la surface de contact des os (54 ; 54') présente un contour correspondant au moins à une partie de la fosse (56) et/ou une partie de l'incisure de l'acétabulum (58).

2. Ensemble d'instruments médicaux selon la revendication 1, **caractérisé en ce que** ledit au moins un bord visible (60, 62, 64 ; 60', 62' ; 108) présente une largeur ou une hauteur correspondant au moins à une épaisseur minimale du corps moulé (18 ; 18' ; 18").

3. Ensemble d'instruments médicaux selon la revendication 2, **caractérisé en ce que** ledit au moins un bord visible (60, 62, 64 ; 60', 62' ; 108) présente une largeur ou une hauteur correspondant au plus au triple d'une épaisseur minimale du corps moulé (18 ; 18' ; 18").

4. Ensemble d'instruments médicaux selon l'une quelconque des revendications précédentes, **caractérisé en ce que** ledit au moins un bord visible (60, 62, 64 ; 60', 62' ; 108) forme une zone de surface plane ou sensiblement plane ou forme une surface de délimitation d'une cavité (112 ; 112' ; 112") du corps moulé (18 ; 18' ; 18"), laquelle cavité (112 ; 112' ; 112") pénétrant au moins partiellement dans la surface de contact des os (54 ; 54').

5. Ensemble d'instruments médicaux selon la revendication 4, **caractérisé en ce que** ladite au moins une cavité (112 ; 112' ; 112") prend la forme d'un jour (100) et **en ce que** ledit au moins un bord visible (108) forme une surface interne (106) du jour (100).

6. Ensemble d'instruments médicaux selon la revendication 5, **caractérisé**
a) **en ce que** l'instrument d'alignement (16) définit un axe longitudinal d'instrument (28) et en ce qu'un axe longitudinal (102) du jour (100) est parallèle ou sensiblement parallèle à l'axe longitudinal d'instrument (28) et/ou
b) **en ce que** le jour (100) forme un trou (104).

7. Ensemble d'instruments médicaux selon l'une quelconque des revendications précédentes, **caractérisé**
a) **en ce que** la surface de contact des os (54 ; 54') est au moins partiellement ajustée à un contour de l'acétabulum (12) du patient
et/ou
b) **en ce que**, sur le corps moulé (18 ; 18"), il existe au moins un logement d'élément de fixation (90) placé ou formé et/ou
c) **en ce que** la surface de contact des os (54 ; 54') et/ou le corps moulé est fabriqué(e) par moulage ou impression en 3D
et/ou
d) **en ce que** la surface de contact des os (54 ; 54') du corps moulé (18 ; 18' ; 18") définit les données de contour de la surface de contact des os qui correspondent ou correspondent sensiblement aux données de contour des os définies de manière non invasive du patient, en particulier aux données de contour des os du patient des radiographies ou des clichés de résonance magnétique.

8. Ensemble d'instruments médicaux selon l'une quelconque des revendications précédentes, **caractérisé par** un élément de serrage (20).

9. Ensemble d'instruments médicaux selon la revendication 8, **caractérisé en ce que** l'élément de serrage (20) peut être raccordé de manière lâche au corps moulé (18 ; 18' ; 18").

10. Ensemble d'instruments médicaux selon l'une quelconque des revendications précédentes, **caractérisé par** un dispositif de couplage (82) pour un couplage temporaire du corps moulé (18 ; 18' ; 18") avec l'élément de serrage (20).

11. Ensemble d'instruments médicaux selon la revendication 10, **caractérisé en ce que** le dispositif de couplage (82) comprend au moins un premier élément de couplage (78) et au moins un second élément de couplage (80), **en ce que** lesdits au moins un premier et un second élément de couplage (78, 80) correspondant l'un à l'autre sont formés, et s'engagent l'un dans l'autre en position de couplage et se dégagent en position de desserrage.

12. Ensemble d'instruments médicaux selon la revendication 11, **caractérisé**
a) **en ce que** ledit au moins un premier élément de couplage (78) prend la forme d'un logement de couplage (74) et ledit au moins un second élément de couplage (80) prend la forme d'une saillie de couplage (76)
et/ou
b) **en ce que** ledit au moins un premier élément de couplage (78) comprend un segment de filetage interne (72) et ledit au moins un second élément de couplage (80) comprend un segment de filetage externe (50) correspondant et/ou
c) **en ce que** ledit au moins un premier élément de couplage (78) est placé ou formé sur le corps moulé (18 ; 18' ; 18") et ledit au moins un second élément de couplage (80) est placé ou formé sur l'élément de serrage (20).

13. Ensemble d'instruments médicaux selon l'une quelconque des revendications 8 à 12, **caractérisé en ce que** l'élément de serrage (20) comprend un arbre (24) s'étendant sur la longueur et **en ce qu'**une zone de serrage (26) est placée ou formée sur une zone d'extrémité proximale de l'arbre (24).

14. Ensemble d'instruments médicaux selon l'une quelconque des revendications 13, **caractérisé**
a) **en ce que** l'arbre (24) définit un axe longitudinal (28) et en ce qu'une surface d'extrémité proximale de l'arbre (24) forme une surface de percussion (42) pour un outil de butée
et/ou
b) **en ce que** l'arbre (24) se présente au moins en deux parties et comprend un manchon (32) et un arbre interne (34) inséré dedans et en ce que le second élément de couplage (80) est placé ou formé sur une extrémité distale (46) de l'arbre interne (34) ou sur une extrémité distale du manchon.

15. Ensemble d'instruments médicaux selon l'une quelconque des revendications précédentes, **caractérisé par** un outil de butée.
